(19) Europäisches Patentamt
European Patent Office
Office européen des brevets

(11) Veröffentlichungsnummer: **0 146 105**
**A2**

(12) **EUROPÄISCHE PATENTANMELDUNG**

(21) Anmeldenummer: **84115118.6**

(22) Anmeldetag: **10.12.84**

(51) Int. Cl.⁴: **C 07 D 249/08**, C 07 D 233/56, C 07 D 403/12, C 07 D 405/12, A 01 N 43/653, A 01 N 43/50

(30) Priorität: **20.12.83 DE 3345899**

(43) Veröffentlichungstag der Anmeldung: **26.06.85**
**Patentblatt 85/26**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Gayer, Herbert, Dr., Alfred-Delp-Strasse 4, D-4019 Monheim (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen (DE)**
Erfinder: **Hänssler, Gerd, Dr., Heymannstrasse 40, D-5090 Leverkusen 1 (DE)**

(54) **Iminomethyl-azolyl-Derivate.**

(57) Die Erfindung betrifft neue Iminomethyl-azolyl-Derivate, mehrere Verfahren zu ihrer Herstellung sowie ihre Verwendung als Pflanzenschutzmittel.

Die Verbindungen der allgemeinen Formel

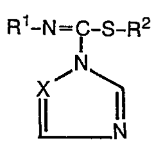

(I)

in welcher X, $R^1$ und $R^2$ die in der Beschreibung genannte Bedeutung besitzen, werden unter anderem erhalten, wenn man Isocyanid-dihalogenide mit Imidazolen oder Triazolen und anschließend mit Thiolen in Gegenwart eines Säureakzeptors und eines Verdünnungsmittels umsetzt.

Die neuen Verbindungen sind für den Gebrauch als Pflanzenschutzmittel geeignet und können mit gutem Erfolg zur Bekämpfung von Pflanzenkrankheiten im Reisanbau, in Getreidekulturen und im Obstbau eingesetzt werden.

EP 0 146 105 A2

BAYER AKTIENGESELLSCHAFT 5090 Leverkusen, Bayerwerk
Konzernverwaltung RP
Patentabteilung Slr/Ja
Ib

## Iminomethyl-azolyl-Derivate

Die Erfindung betrifft neue Iminomethyl-azolyl-Derivate, mehrere Verfahren zu ihrer Herstellung sowie ihre Verwendung als Pflanzenschutzmittel.

Es ist bereits bekannt, daß bestimmte Imidazolyl-amidine, wie beispielsweise 1-(3,4-Dichlorphenyl)-1-methyl-2-imidazol-1-yl-3-i-propyl-amidin, 1-Phenyl-1-trifluormethyl-2-(imidazol-1-yl)-3-phenyl-amidin, 1,3-Dimethyl-2-(imidazol-1-yl)-1-phenyl-amidin und 1-(2,4-Dichlorphenyl)-1-ethyl-2-(imidazol-1-yl)-3-i-propyl-amidin gute fungizide Eigenschaften aufweisen (vgl. z.B. DE-OS 25 49 899, ähnliche Verbindungen sind auch aus den offengelegten japanischen Patentanmeldungen Nr. 65 879/1978 und 132 567/1979 bekannt). Die Wirkung dieser Verbindungen ist jedoch, insbesondere bei niedrigen Aufwandmengen und -konzentrationen nicht immer ganz befriedigend.

Es wurden nun neue Iminomethyl-azolyl-Derivate der allgemeinen Formel (I)

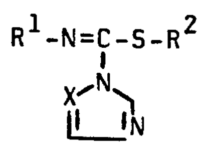

(I)

in welcher

X für Stickstoff oder eine CH-Gruppe steht,

$R^1$ für geradkettiges oder verzweigtes Alkyl, Alkenyl, Alkoxyalkyl, gegebenenfalls substituiertes Cycloalkyl, gegebenenfalls im Phenylteil substituiertes Phenyl, Phenylalkyl oder Phenoxyalkyl steht, und

$R^2$ für gegebenenfalls substituiertes, geradkettiges oder verzweigtes Alkyl, für Alkenyl, Alkinyl, Halogenalkyl, Halogenalkenyl, Cyano, gegebenenfalls substituiertes Benzyl oder Phenyl, sowie für einen gegebenenfalls durch Sauerstoff und/oder eine Ketogruppe unterbrochenen Cycloalkyl-Rest steht,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen,

und deren Säureadditionssalze und Metallsalz-Komplexe gefunden.

Weiterhin wurde gefunden, daß man die neuen Iminomethyl-azolyl-Derivate der Formel (I) erhält, wenn man

a) für den Fall, daß X für eine CH-Gruppe steht, Senföle der Formel (II)

$$R^1\text{-N=C=S} \qquad \text{(II)}$$

in welcher

$R^1$ die oben angegebene Bedeutung hat,

mit Imidazol, in Gegenwart einer starken Base und gegebenenfalls in Gegenwart eines Verdünnungsmittels umsetzt, und die auf diese Weise erhaltenen Salze der Formel (III)

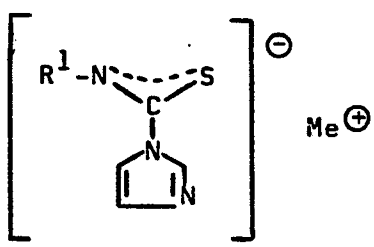

(III)

in welcher

$R^1$ die oben angegebene Bedeutung hat und

Me für ein Äquivalent eines Alkali- oder Erdalkalimetallatoms steht,

gegebenenfalls isoliert und mit Halogeniden der Formel (IV)

$$R^2Hal \qquad (IV)$$

in welcher

$R^2$ die oben angegebene Bedeutung hat und

Hal für Halogen steht,

gegebenenfalls in Gegenwart eines Verdünnungsmittels umsetzt;

oder wenn man

b) für den Fall, daß X für eine CH-Gruppe oder für Stickstoff steht,

Isocyanid-dihalogenide der Formel (V)

$$R^1-N=C(Hal^1)_2 \qquad (V)$$

in welcher

$R^1$ die oben angegebene Bedeutung hat, und

$Hal^1$ für Halogen, wie Chlor oder Brom steht,

mit Imidazol bzw. Triazol, in Gegenwart eines Säureakzeptors und gegebenenfalls in Gegenwart eines Verdünnungsmittels umsetzt und die auf diese Weise erhaltenen Verbindungen der Formel (VI)

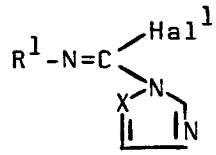

(VI)

in welcher

$R^1$, X und $Hal^1$ die oben angegebenen Bedeutungen haben,

gegebenenfalls isoliert und mit Thiolen der Formel (VII)

$$R^2S-Y \qquad (VII)$$

in welcher

$R^2$ die oben angegebene Bedeutung hat und

Y für Wasserstoff oder ein Aequivalent eines Alkali- oder Erdalkali-metallatoms steht,

gegebenenfalls in Gegenwart eines Säureakzeptors und gegebenenfalls in Gegenwart von Verdünnungsmitteln umsetzt.

An die auf diese Weise erhaltenen Verbindungen der Formel (I) können gegebenenfalls anschließend eine Säure oder ein Metallsalz addiert werden.

Die erfindungsgemäßen Verbindungen zeigen überraschenderweise eine höhere fungizide Wirkung als die bekannten Wirkstoffe 1-(3,4-Dichlorphenyl)-1-methyl-2-imidazol-1-yl-3-i-propyl-amidin, 1-Phenyl-1-trifluormethyl-2-(imidazol-1-yl)-3-phenyl-amidin, 1,3-Dimethyl-2-(imidazol-1-yl)-1-phenyl-amidin und 1-(2,4-Dichlorphenyl)-1-ethyl-2-(imidazol-1-yl)-3-i-propyl-amidin. Die erfindungsgemäßen Wirkstoffe stellen somit eine Bereicherung der Technik dar.

Die erfindungsgemäßen Iminomethyl-azolyl-Derivate sind durch die Formel (I) allgemein definiert.

Bevorzugt sind diejenigen Verbindungen der Formel (I), in denen

X für Stickstoff oder eine CH-Gruppe steht,

$R^1$ für geradkettiges oder verzweigtes Alkyl und Alkoxyalkyl mit jeweils 1 bis 12 Kohlenstoffatomen in den Alkylteilen, für Alkenyl mit bis zu 4 Kohlenstoffatomen, für gegebenenfalls einfach oder mehrfach, gleich oder verschieden durch Halogen und/oder Alkyl substituiertes Cycloalkyl mit 3 bis 8 Kohlenstoffatomen, für gegebenenfalls einfach oder mehrfach, gleich oder verschieden im Phenylteil substituiertes Phenyl, Phenylalkyl und Phenoxyalkyl, mit jeweils 1 bis 6 Kohlenstoffatomen im Alkylteil steht, wobei als jeweilige Phenylsubstituenten bevorzugt infrage kommen:

Halogen, wie insbesondere Fluor, Chlor, Brom und/oder Iod; Alkyl, Alkoxy und Alkylthio mit jeweils 1 bis 4 Kohlenstoffatomen im Alkylteil; Halogenalkyl, Halogenalkoxy und Halogenalkylthio mit jeweils 1 bis 4 Kohlenstoffatomen im Alkylteil und 1 bis 5 gleichen

oder verschiedenen Halogenatomen, wie Fluor, Chlor und/ oder Brom und/oder Phenoxy;

$R^2$ für gegebenenfalls einfach oder mehrfach, gleich oder verschieden substituiertes, geradkettiges oder verzweigtes Alkyl mit 1 bis 12 Kohlenstoffatomen steht, wobei als Alkylsubstituenten bevorzugt infrage kommen :

ein Phthalimido-Rest; Cyano; Halogen, wie Fluor, Chlor, Brom und/oder Iod; eine gegebenenfalls durch Sauerstoff unterbrochene Cycloalkylgruppierung mit 2 bis 6 Kohlenstoffatomen; und/oder die Gruppierungen $R^3O-$, $R^3S-$, $R^3-CO-O$, $R^3O-CO$, $R^3-CO-$, $R^3O-CO-(CH_2)_n-CO-$, $(R^4O)(R^5O)-CR^6$ und $R^7R^8N-CO-$, in welcher

$R^3$, $R^4$ und $R^5$ gleich oder verschieden sind und für geradkettiges oder verzweigtes Alkyl mit 1 bis 6 Kohlenstoffatomen oder für gegebenenfalls durch Fluor, Chlor, Methyl, Methoxy, Methylthio, Trifluormethyl, Trifluormethoxy und/oder Trifluormethylthio substituiertes Phenyl stehen, oder

$R^4$ und $R^5$ gemeinsam für einen gegebenenfalls durch Fluor und/oder Chlor substituierten Alkandiyl-Rest mit 2 oder 3 Kohlenstoffatomen stehen,

n für die Zahlen 0 oder 1 steht,

$R^6$, $R^7$ und $R^8$ gleich oder verschieden sind und für Wasserstoff, geradkettiges oder verzweigtes Alkyl mit 1 bis 4 Kohlenstoffatomen oder für gegebenenfalls durch Fluor, Chlor, Methyl, Methoxy, Methylthio, Trifluormethyl, Trifluormethoxy und/oder Trifluormethylthio substituiertes Phenyl stehen;

$R^2$ steht weiterhin vorzugsweise für Alkenyl und Alkinyl mit 3 bis 6 Kohlenstoffatomen; für Halogenalkenyl mit 3 bis 6 Kohlenstoffatomen im Alkenylteil und 1 bis 3 Halogenatomen, wie insbesondere Chlor, Brom und/oder Iod; für Cyano oder für gegebenenfalls substituiertes Benzyl oder Phenyl, wobei als Phenylsubstituenten vorzugsweise die Substituenten infrage kommen, die bei der Beschreibung von $R^1$ als Phenylsubstituenten genannt worden sind; sowie für eine gegebenenfalls durch Sauerstoff und/oder eine Ketogruppe unterbrochene Cycloalkylgruppe mit 3 bis 6 Kohlenstoffatomen,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen.

Besonders bevorzugt sind diejenigen Verbindungen der Formel (I), in denen

X für Stickstoff oder eine CH-Gruppe steht,

$R^1$ für Methyl, Ethyl, n-Propyl,i-Propyl, n-Butyl, i-Butyl, sec.-Butyl, tert.-Butyl, Pentyl, Hexyl, Heptyl, Octyl, Nonyl, Decyl, Dodecyl, Methoxymethyl, Methoxyethyl, Methoxy-n-propyl, Methoxy-i-propyl, Methoxy-n-butyl, Methoxy-i-butyl, Ethoxymethyl, Ethoxyethyl, Ethoxy-n-propyl, Ethoxy-i-propyl, Ethoxy-n-butyl, Ethoxy-i-butyl, n-Propoxymethyl, n-Propoxyethyl, n-Propxoy-n-propyl, n-Propoxy-i-propyl, n-Propoxy-n-butyl, n-Propoxy-i-butyl, i-Propoxymethyl, i-Propoxyethyl, i-Propoxy-n-propyl, i-Propoxy-i-propyl, i-Propoxy-n-butyl, i-Propoxy-i-butyl, n-Butoxymethyl, n-Butoxyethyl, n-Butoxy-n-propyl, n-Butoxy-i-propyl, n-Butoxy-n-butyl, n-Butoxy-i-butyl, i-Butoxymethyl, i-Butoxyethyl, i-Butoxy-n-propyl, i-Butoxy-i-propyl, i-Butoxy-n-butyl, i-Butoxy-i-butyl, Cyclopropyl, Cyclopentyl, Cyclohexyl, für gegebenenfalls einfach bis dreifach, gleich oder verschieden durch Fluor, Chlor, Brom, Methyl, Ethyl, n-Propyl, i-Propyl, n-Butyl, i-Butyl, sec.-Butyl und/oder tert.-Butyl substituiertes Cyclohexyl, für gegebenenfalls einfach bis dreifach, gleich oder verschieden im Phenylteil durch Fluor, Chlor, Brom, Methyl, Ethyl, n-Propyl, i-Propyl, n-Butyl, i-Butyl, sec.-Butyl, tert.-Butyl, Methoxy, Ethoxy,n-Propoxy, i-Propoxy, n-Butoxy, i-Butoxy, sec.-Butoxy, tert.-Butoxy, Methylthio, Ethylthio, n-Propylthio, i-Propylthio, n-Butylthio, i-Butylthio, sec.-Butylthio, tert.-Butylthio, Phenoxy, Trifluormethyl, Trifluormethoxy und/oder Trifluormethylthio substituiertes Phenyl, Benzyl, Phenylethyl, Benzyloxy und Phenylethoxy steht und

$R^2$ für gegebenenfalls einfach bis zweifach substituiertes Methyl, Ethyl, n-Propyl, i-Propyl, n-Butyl, i-Butyl, sec.-Butyl, tert.-Butyl, Pentyl, Hexyl, Heptyl, Octyl, Nonyl, Decyl und Dodecyl steht, wobei als Substituenten bevorzugt infrage kommen :
ein Phthalimido-Rest, Cyano, Fluor, Chlor, Brom, Cyclopentyl, Cyclohexyl, 2-Oxiranyl, 2-Tetrahydrofuranyl, 2-Tetrahydropyranyl und/oder die Gruppierungen $R^3O-$, $R^3S-$, $R^3-CO-O-$, $R^3O-CO-$, $R^3-CO-$, $R^3O-CO-(CH_2)_n-CO-$, $(R^4O)(R^5O)CR^6-$ und $R^7R^8N-CO-$, in welcher

$R^3$, $R^4$ und $R^5$ gleich oder verschieden sind und für Methyl, Ethyl, n-Propyl, i-Propyl, n-Butyl, i-Butyl, sec.-Butyl, tert.-Butyl oder für gegebenenfalls durch Fluor, Chlor, Methyl, Methoxy, Trifluormethyl und/oder Trifluormethoxy substituiertes Phenyl stehen, oder

$R^4$ und $R^5$ gemeinsam für einen Alkandiyl-Rest mit 2 oder 3 Kohlenstoffatomen stehen,

n für die Zahlen 0 oder 1 steht,

$R^6$, $R^7$ und $R^8$ gleich oder verschieden sind und für Wasserstoff, Methoxy, Ethyl, n-Propyl, i-Propyl, n-Butyl, i-Butyl, sec.-Butyl, tert.-Butyl und für gegebenenfalls durch Fluor, Chlor, Methyl, Methoxy, Trifluormethyl und/oder Trifluormethoxy substituiertes Phenyl stehen;

$R^2$ steht weiterhin für 2-Propen-1-yl, 2-Buten-1-yl, 2-Methyl-2-propen-1-yl, 3-Methyl-2-buten-1-yl, 2-Propin-1-yl, 2-Butin-1-yl, 3-Methyl-2-butin-1-yl,

3-Chlor-2-buten-1-yl, 3-Brom-2-buten-1-yl, Cyano oder für einen gegebenenfalls durch Fluor, Chlor, Brom, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Methylthio, Ethylthio, n-Propylthio, i-Propylthio, n-Butylthio, i-Butylthio, sec.-Butylthio, tert.-Butylthio, Trifluormethyl, Trifluormethoxy und/oder Trifluormethylthio substituiertes Benzyl oder Phenyl, sowie für Cyclohexyl, Cyclopentyl, 2-Oxo-cyclohexyl, 2-Oxo-cyclopentyl, 2-Oxo-tetrahydrofuran-3-yl oder 2-Oxo-tetrahydropyran-3-yl,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen.

Außerdem sind noch besonders bevorzugt diejenigen Verbindungen der allgemeinen Formel (I), bei denen

X für Stickstoff oder die CH-Gruppe steht,

$R^1$ für Alkyl mit 1 bis 12 Kohlenstoffatomen und für Alkenyl mit bis zu 4 Kohlenstoffatomen steht, ferner für Cycloalkyl 5 bis 6 Kohlenstoffatomen steht, wobei letzteres durch 1 bis 3 Alkylgruppen mit bis zu 3 Kohlenstoffatomen substituiert sein kann, und schließlich für Phenyl, Phenylalkyl und Phenoxyalkyl mit jeweils bis zu 4 Kohlenstoffatomen im Alkylteil steht, wobei die drei letztgenannten Gruppen im Phenylteil ein- bis dreifach substituiert sein können durch Halogen, Alkyl, Alkoxy und Alkylthio mit jeweils 1 bis 3 Kohlenstoffatomen, und/oder durch Halogenalkyl mit bis zu 2 Kohlenstoff- und bis zu 5 Halogenatomen, wie insbesondere durch Trifluormethyl, und

$R^2$ für Alkenyl und Alkinyl mit 3 bis 4 Kohlenstoffatomen steht, wobei die beiden letztgenannten Reste 1 bis 3 Halogenatome tragen können, weiterhin für Cyano steht, für Cyclohexylmethyl oder für Phenyl, wobei der letztgenannte Rest durch die bei $R^1$ genannten Phenylsubstituenten substituiert sein kann, und schließlich für eine Cycloalkylgruppe mit 3 bis 6 Kohlenstoffatomen steht, in welche Sauerstoff oder eine Carbonylgruppe in das Ringsystem eingefügt sein kann, weiterhin kann noch für den Fall, daß

a) nicht gleichzeitig
   X für eine CH-Gruppe und
   $R^1$ für durch Halogen und/oder Alkyl substituiertes Phenyl steht,
   $R^2$ noch zusätzlich stehen für primäres, sekundäres

oder tertiäres Alkyl mit bis zu 12 Kohlenstoffatomen steht, welches substituiert sein kann durch Halogen, Cyano, einen Phthalimido-Rest, durch Alkoxy- und Alkylthio-Reste mit jeweils bis zu 3 Kohlenstoffatomen, durch Alkylcarbonyl, Alkoxycarbonyl und Alkylcarbonyloxy mit jeweils bis zu 4 Kohlenstoffatomen in den Alkylteilen, und schließlich kann noch für den Fall, daß

b) nicht gleichzeitig
X für Stickstoff oder eine CH-Gruppe und
$R^1$ für gegebenenfalls durch Halogen, Alkyl, Alkoxy und/oder Trifluormethyl substituiertes Phenyl steht,
$R^2$ noch zusätzlich für Benzyl stehen, welches durch die bei $R^1$ genannten Phenylsubstituenten im Phenylteil substituiert sein kann.

Bevorzugte erfindungsgemäße Verbindungen sind auch Additionsprodukte aus Säuren und denjenigen Iminomethylazolyl-Derivaten der Formel (I), in denen die Substituenten $R^1$, $R^2$ und X die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Iminomethyl-azolyl-Derivaten der Formel (I), in denen die Substituenten $R^1$, $R^2$ und X die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden Verbindungen der allgemeinen Formel (I) genannt :

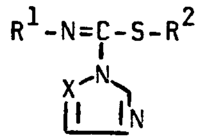 (I)

Tabelle 1.1

X = CH

| $R^1$ | $R^2$ | $R^1$ | $R^2$ |
|---|---|---|---|
| 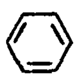 | $H_5C_2O-CH_2CH_2-$ | 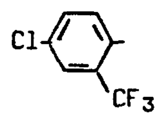 | $H_3CS-CH_2CH_2-$ |
| 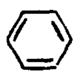 | $H_3CO-CH_2CH_2-$ | 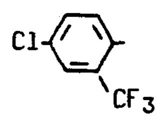 | $H_2C=CH-CH_2-$ |
| 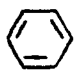 | $H_5C_2S-CH_2CH_2-$ | 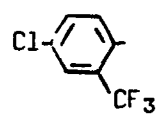 | $H_3C-CH=CH-CH_2-$ |
| 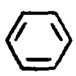 | $H_3CS-CH_2CH_2-$ | 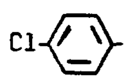 | $H_5C_2O-CH_2CH_2-$ |
| 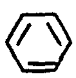 | $H_2C=CH-CH_2-$ | 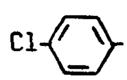 | $H_3CO-CH_2CH_2-$ |
| 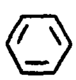 | $H_3C-CH=CH-CH_2-$ | 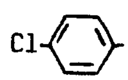 | $H_5C_2S-CH_2CH_2-$ |
| 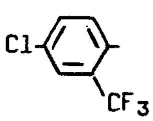 | $H_5C_2O-CH_2CH_2-$ | 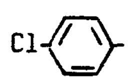 | $H_3CS-CH_2CH_2-$ |
| 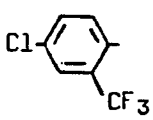 | $H_3CO-CH_2CH_2-$ | 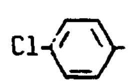 | $H_2C=CH-CH_2-$ |
| 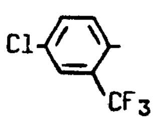 | $H_5C_2S-CH_2CH_2-$ | 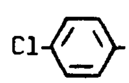 | $H_3C-CH=CH-CH_2-$ |

0146105

Fortsetzung Tabelle 1.1

| $R^1$ | $R^2$ | $R^1$ | $R^2$ |
|---|---|---|---|
| 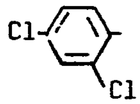 | $H_5C_2O-CH_2-CH_2-$ | 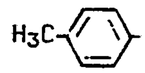 | $H_5C_2O-CH_2CH_2-$ |
| 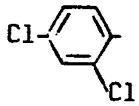 | $H_3CO-CH_2CH_2-$ | 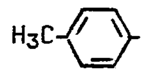 | $H_3CO-CH_2CH_2-$ |
| 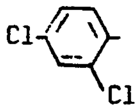 | $H_5C_2S-CH_2CH_2$ | 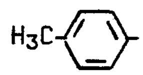 | $H_5C_2S-CH_2CH_2-$ |
| 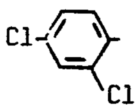 | $H_3CS-CH_2CH_2-$ | 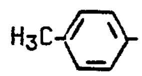 | $H_3CS-CH_2CH_2-$ |
| 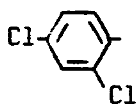 | $H_3C-CH=CH-CH_2-$ | 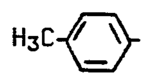 | $H_2C=CH-CH_2-$ |
| 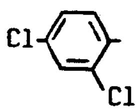 | $H_2C=CH-CH_2-$ | 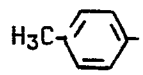 | $H_3C-CH=CH-CH_2-$ |
| 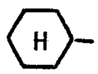 | $H_5C_2O-CH_2CH_2-$ | 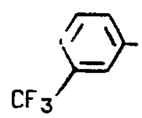 | $H_5C_2O-CH_2CH_2-$ |
| 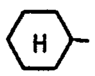 | $H_3CO-CH_2CH_2-$ | 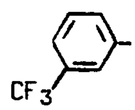 | $H_3CO-CH_2CH_2-$ |
| 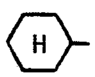 | $H_5C_2S-CH_2CH_2-$ | 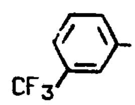 | $H_5C_2S-CH_2CH_2-$ |
| 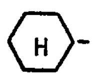 | $H_3CS-CH_2CH_2-$ | 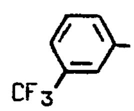 | $H_3CS-CH_2CH_2-$ |
| 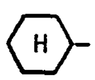 | $H_3C-CH=CH-CH_2-$ | 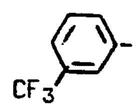 | $H_2C=CH-CH_2-$ |

Fortsetzung Tabelle 1.1

| $R^1$ | $R^2$ | $R^1$ | $R^2$ |
|---|---|---|---|
| 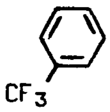 | $H_3C-CH=CH-CH_2-$ | 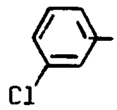 | $H_3C-CH=CH-CH_2-$ |
| 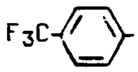 | $H_5C_2O-CH_2CH_2-$ | 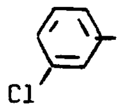 | $H_2C=CH-CH_2-$ |
| 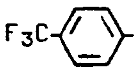 | $H_3CO-CH_2CH_2-$ | 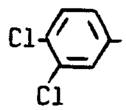 | $H_5C_2O-CH_2CH_2-$ |
| 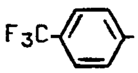 | $H_5C_2S-CH_2CH_2-$ | 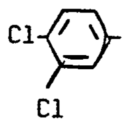 | $H_3CO-CH_2CH_2-$ |
| 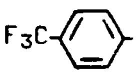 | $H_3CS-CH_2CH_2-$ | 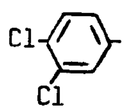 | $H_5C_2S-CH_2CH_2-$ |
| 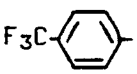 | $H_3C-CH=CH-CH_2-$ | 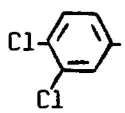 | $H_3CS-CH_2CH_2-$ |
| 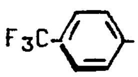 | $H_2C=CH-CH_2-$ | 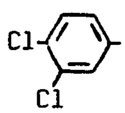 | $H_3C-CH=CH-CH_2-$ |
| 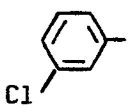 | $H_5C_2O-CH_2CH_2-$ | 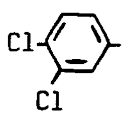 | $H_2C=CH-CH_2-$ |
| 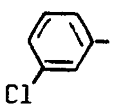 | $H_3CO-CH_2CH_2-$ | 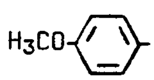 | $H_5C_2O-CH_2CH_2-$ |
| 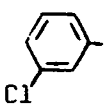 | $H_5C_2S-CH_2CH_2-$ | | |
| 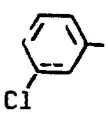 | $H_3CS-CH_2CH_2-$ | | |

Fortsetzung Tabelle 1.1

| $R^1$ | $R^2$ | $R^1$ | $R^2$ |
|---|---|---|---|
| 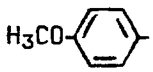 | $H_3CO-CH_2CH_2-$ | 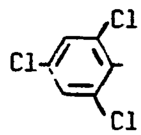 | $H_3C-CH=CH-CH_2-$ |
| 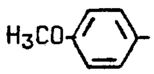 | $H_5C_2S-CH_2CH_2-$ | 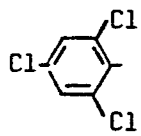 | $H_2C=CH-CH_2-$ |
| 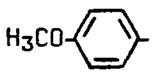 | $H_3CS-CH_2CH_2-$ | 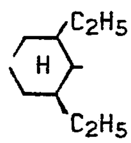 | $H_5C_2O-CH_2CH_2-$ |
| 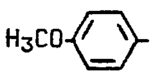 | $H_3C-CH=CH-CH_2-$ | 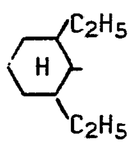 | $H_3CO-CH_2CH_2-$ |
| 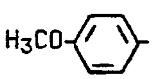 | $H_2C=CH-CH_2-$ | 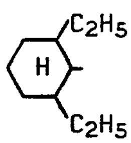 | $H_5C_2S-CH_2CH_2-$ |
| 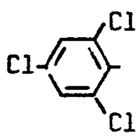 | $H_5C_2O-CH_2CH_2-$ | 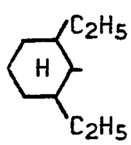 | $H_3CS-CH_2CH_2-$ |
| 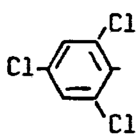 | $H_3CO-CH_2CH_2-$ | 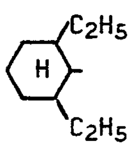 | $H_3C-CH=CH-CH_2-$ |
| 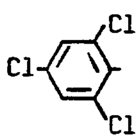 | $H_5C_2S-CH_2CH_2-$ | 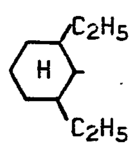 | $H_2C=CH-CH_2-$ |
| 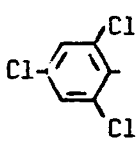 | $H_3CS-CH_2CH_2-$ | | |

Tabelle 1.2

X = N

| $R^1$ | $R^2$ | $R^1$ | $R^2$ |
|---|---|---|---|
| 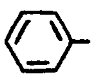 | $-CH_3$ | 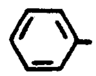 | 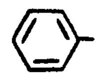 |
| 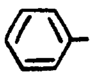 | $-C_2H_5$ | 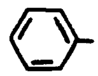 | 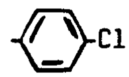 |
| 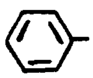 | $-C_3H_7$-n | 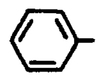 | 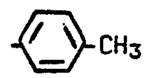 |
| 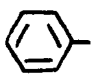 | $-C_3H_7$-i | 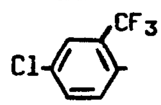 | $-CH_3$ |
| 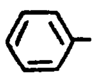 | $-C_4H_9$-n | 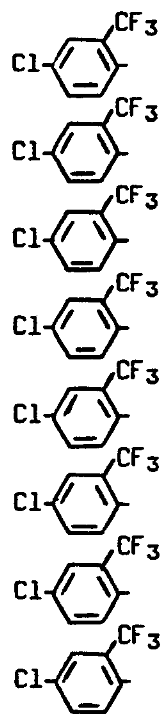 | $-C_2H_5$ |
| 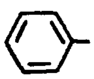 | $-C_4H_9$-tert | | $-C_3H_7$-n |
| 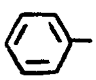 | $-C_5H_{11}$-n | | $C_3H_7$-i |
| 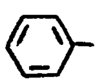 | $-C_6H_{13}$-n | | $C_4H_9$-n |
| 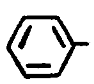 | $-C_8H_{17}$-n | | $C_4H_9$-tert |
| 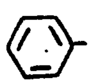 | $-C_{12}H_{25}$-n | | $-C_5H_{11}$-n |
| 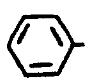 | $-CH_2-CH=CH_2$ | | $-C_6H_{13}$-n |
| 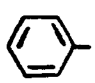 | 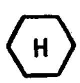 | | $C_8H_{17}$-n |

Fortsetzung Tabelle 1.2

| $R^1$ | $R^2$ | $R^1$ | $R^2$ |
|---|---|---|---|
| 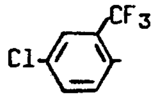 | -$C_{12}H_{25}$-n | 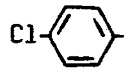 | -$C_8H_{17}$-n |
| 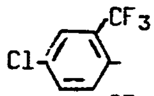 | -$CH_2$-CH=$CH_2$ | 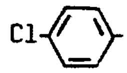 | -$C_{12}H_{25}$-n |
| 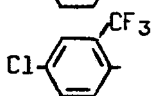 | 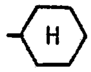 | 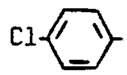 | -$CH_2$-CH=$CH_2$ |
| 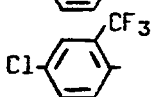 | 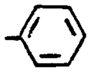 | 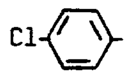 | 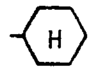 |
| 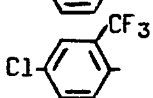 | 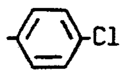 | 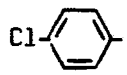 | 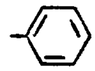 |
| 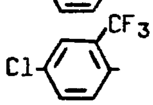 | 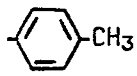 | 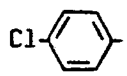 | 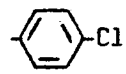 |
| 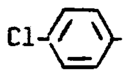 | -$CH_3$ | 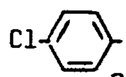 | 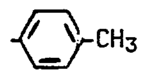 |
| 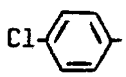 | -$C_2H_5$ | 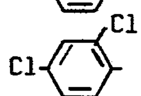 | -$CH_3$ |
| 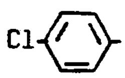 | -$C_3H_7$-n | 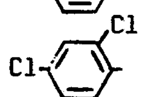 | -$C_2H_5$ |
| 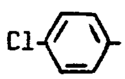 | -$C_3H_7$-i | 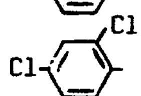 | -$C_3H_7$-n |
| 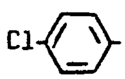 | -$C_4H_9$-n | 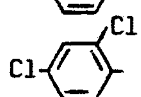 | -$C_3H_7$-i |
| 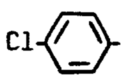 | $C_4H_9$-tert. | 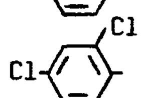 | -$C_4H_9$-n |
| 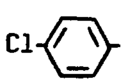 | -$C_5H_{11}$-n | 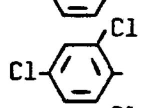 | -$C_4H_9$-tert. |
| 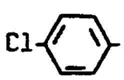 | -$C_6H_{13}$-n | 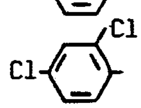 | -$C_5H_{11}$-n |

Fortsetzung Tabelle 1.2

| $R^1$ | $R^2$ | $R^1$ | $R^2$ |
|---|---|---|---|
| 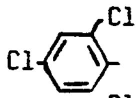 | $C_6H_{13}$-n | 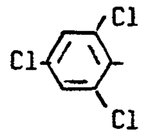 | $-C_4H_9$-n |
| 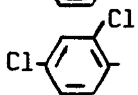 | $-C_8H_{17}$-n | 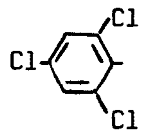 | $-C_4H_9$-tert. |
| 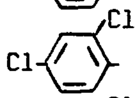 | $-C_{12}H_{25}$-n | 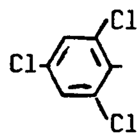 | $-C_5H_{11}$-n |
| 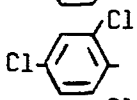 | $-CH_2-CH=CH_2$ | 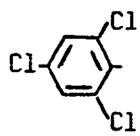 | $-C_6H_{13}$-n |
| 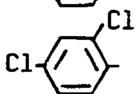 | 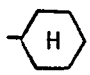 | 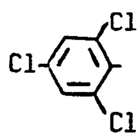 | $-C_8H_{17}$-n |
| 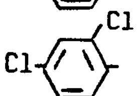 | 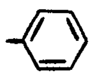 | 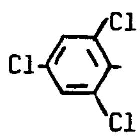 | $-C_{12}H_{25}$-n |
| 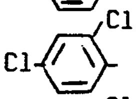 | 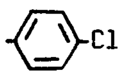 | 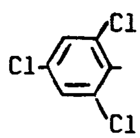 | $-CH_2-CH=CH_2$ |
| 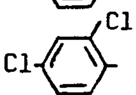 | 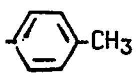 | 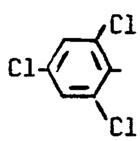 | 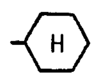 |
| 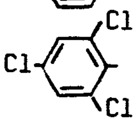 | $-CH_3$ | 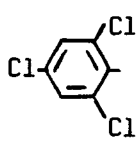 | 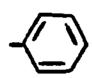 |
| 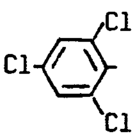 | $-C_2H_5$ | | |
| 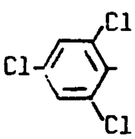 | $-C_3H_7$-n | | |
| 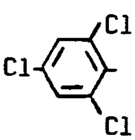 | $-C_3H_7$-i | | |

Fortsetzung Tabelle 1.2

| $R^1$ | $R^2$ | $R^1$ | $R^2$ |
|---|---|---|---|
| 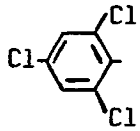 | 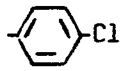 | 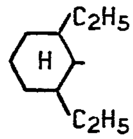 | $-C_6H_{13}-n$ |
| 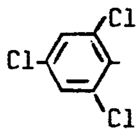 | 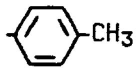 | 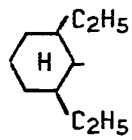 | $-C_8H_{17}-n$ |
| 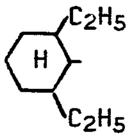 | $-CH_3$ | 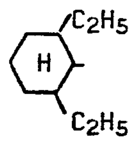 | $-C_{12}H_{25}-n$ |
| 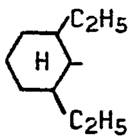 | $-C_2H_5$ | 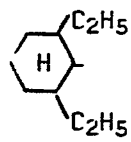 | $-CH_2-CH=CH_2$ |
| 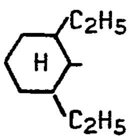 | $-C_3H_7-n$ | 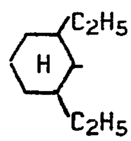 | 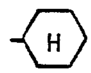 |
| 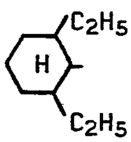 | $-C_3H_7-i$ | 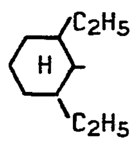 | 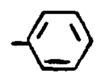 |
| 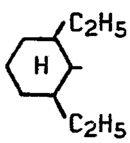 | $-C_4H_9-n$ | 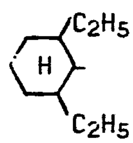 | 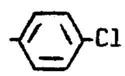 |
| 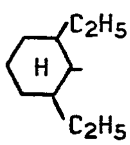 | $-C_4H_9-tert.$ | 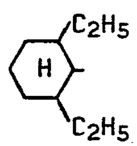 | 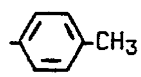 |
| 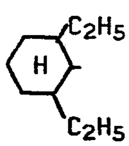 | $-C_5H_{11}-n$ | | |

Verwendet man beispielsweise Phenylsenföl, Imidazol und Ethoxyethylchlorid als Ausgangsstoffe für das Verfahren (a), so kann der Reaktionsablauf durch das folgende Formelschema wiedergegeben werden :

(a)

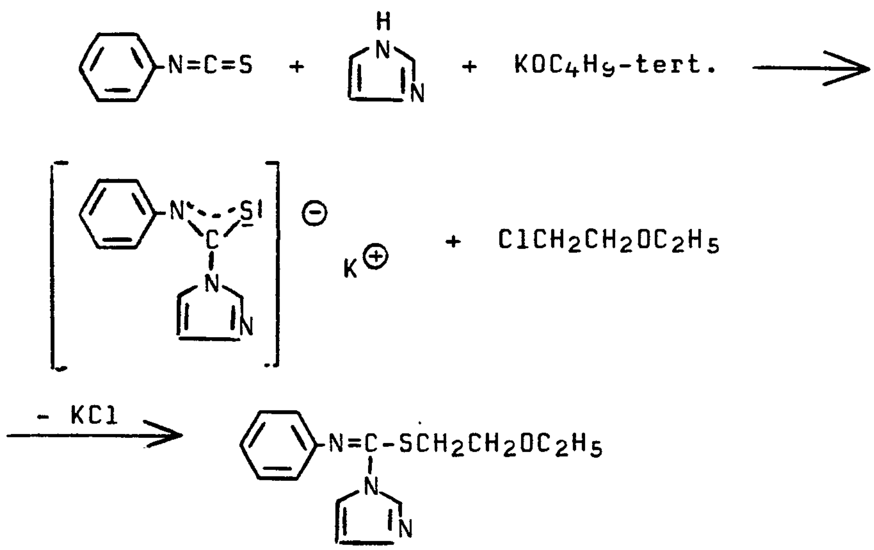


Verwendet man beispielsweise 4-Chlorphenylisocyaniddichlorid, Triazol und n-Butanthiol als Ausgangsstoffe für das erfindungsgemäße Verfahren (b), so kann der Reaktionsablauf durch das folgende Formelschema wiedergegeben werden :

(b)

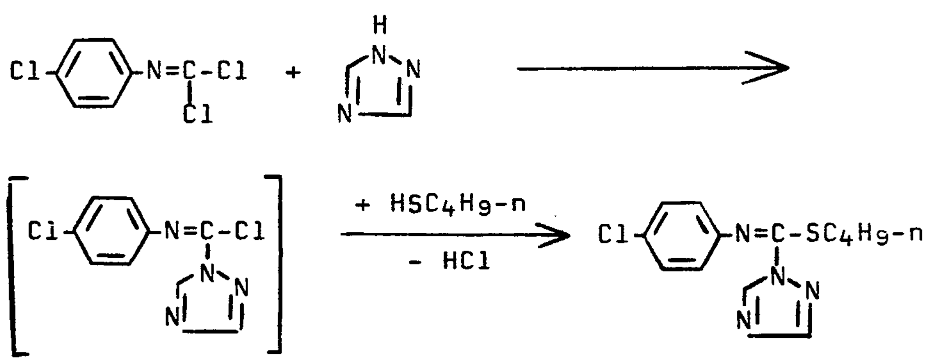

Die bei der Durchführung des erfindungsgemäßen Verfahrens (a) als Ausgangsstoffe benötigten Senföle sind durch die Formel (II) allgemein definiert. In dieser Formel hat $R^1$ die Bedeutung, die bereits im Zusammenhang mit der Beschreibung der erfindungsgemäßen Stoffe der Formel (I) vorzugsweise für diese Substituenten genannt wurden.

Die Senföle der Formel (II) sind bekannte Verbindungen der organischen Chemie (vgl. z.B. Houben-Weyl-Müller, 4. Auflage, Band IX, Seite 867 ff., Georg Thieme Verlag, Stuttgart, New York).

Die Salze, die bei der Durchführung des erfindungsgemäßen Verfahrens (a) als Zwischenprodukte entstehen und gegebenenfalls isoliert werden können, sind durch die Formel (III) allgemein definiert. In dieser Formel hat $R^1$ die Bedeutung, die bereits im Zusammenhang mit der Beschreibung der erfindungsgemäßen Stoffe der Formel (I) vorzugsweise für diesen Substituenten genannt wurde. Me steht in dieser Formel für ein Aequivalent eines Alkali- oder Erdalkali-metallatoms wie Natrium, Kalium, Lithium, Magnesium oder Calcium, vorzugsweise für Natrium oder Kalium.

Die Verbindungen der Formel (III) sind neu und können nach an sich bekannten Methoden hergestellt werden, indem man z.B. Imidazol umsetzt mit Senfölen der Formel (II)

$$R^1\text{-N=C=S} \qquad \text{(II)}$$

in welcher

$R^1$ die oben angegebene Bedeutung hat,

in Gegenwart einer starken Base, wie z.B. Kalium-tert.-butylat, und gegebenenfalls in Gegenwart von Verdünnungsmitteln, wie z.B. Tetrahydrofuran, bei Temperaturen zwischen -20°C und +150°C, vorzugsweise zwischen 0°C und 100°C. Die auf diese Weise anfallenden Verbindungen der Formel (III) können nach üblichen Methoden isoliert werden oder lassen sich mit den Halogeniden der Formel (IV) gemäß Verfahren (a) weiter zu den erfindungsgemäßen Verbindungen der Formel (I) umsetzen.

Die weiterhin für das Verfahren (a) als Ausgangsstoffe zu verwendenden Halogenide sind durch die Formel (IV) allgemein definiert. In dieser Formel hat $R^2$ die Bedeutung, die bereits im Zusammenhang mit der Beschreibung der erfindungsgemäßen Stoffe der Formel (I) vorzugsweise für diesen Substituenten genannt wurde.

Hal steht in dieser Formel für Halogen, wie Chlor, Brom oder Iod, vorzugsweise für Chlor oder Brom.

Die Halogenide der Formel (IV) sind allgemein bekannte Verbindungen der organischen Chemie.

Die bei der Durchführung des erfindungsgemäßen Verfahrens (b) als Ausgangsstoffe benötigten Dihalogenide sind durch die Formel (V) definiert. In dieser Formel hat $R^1$ die Bedeutung, die bereits im Zusammenhang mit der Beschreibung der erfindungsgemäßen Stoffe der Formel (I) vorzugsweise für diesen Substituenten genannt wurde.

$Hal^1$ steht in dieser Formel für Halogen, wie Chlor, Brom oder Iod, vorzugsweise für Chlor oder Brom.

Die Verbindungen der Formel (V) sind allgemein bekannte Verbindungen der organischen Chemie.

Die Zwischenprodukte, die beim erfindungsgemäßen Verfahren (b) gegebenenfalls isoliert werden können, sind durch die Formel (VI) allgemein definiert. In dieser Formel haben $R^1$ und X die Bedeutungen, die bereits im Zusammenhang mit der Beschreibung der erfindungsgemäßen Stoffe der Formel (I) vorzugsweise für diesen Substituenten genannt wurden. $Hal^1$ steht in dieser Formel für Halogen, wie Chlor, Brom oder Iod, vorzugsweise für Chlor oder Brom.

Die Verbindungen der Formel (VI) sind bekannt (vgl. dazu die Derwent-Referate 52 431 A 29 der JP-OS 53 065 879 und 85 103 B 47 der JP-OS 54 132 567).

Die weiterhin als Ausgangsstoffe für das Verfahren (b) zu verwendenden Thiole sind durch die Formel (VII) allgemein definiert. In dieser Formel hat $R^2$ die Bedeutung, die bereits im Zusammenhang mit der Beschreibung der erfindungsgemäßen Stoffe der Formel (I) vorzugsweise für diesen Substituenten genannt wurde.

Y steht in dieser Formel für Wasserstoff oder für ein Aequivalent eines Alkali- oder Erdalkali-metallatoms, wie Natrium, Kalium oder Calcium. Vorzugsweise steht Y für Wasserstoff.

Die Verbindungen der Formel (VII) bzw. ihre Salze sind bekannte Verbindungen der organischen Chemie.

Als Verdünnungsmittel kommen beim erfindungsgemäßen Verfahren (Varianten (a) und (b)) praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Säureakzeptoren für die Verfahrensvarianten (a) und (b) können praktisch alle üblicherweise verwendbaren Säurebindemittel eingesetzt werden. Hierzu gehören insbesondere : Alkali- und Erdalkalihydroxide bzw. -oxide wie Natrium- und Kaliumhydroxid und insbesondere Lithiumhydroxid sowie Calciumoxid oder Calcium-hydroxid, Alkali- und Erdalkali-carbonate, wie Natrium-, Kalium- und Calciumcarbonat, Alkalialkoholate, wie Natrium-methylat, -ethylat und -tert.-butylat, Kaliummethylat, -ethylat und -tert.-butylat, ferner aliphatische, aromatische oder heterocyclische Amine, wie Triethylamin, Dimethylanilin, Dimethylbenzylamin, Pyridin, Diazabicyclooctan und Diazabicycloundecen.

Die Reaktionstemperatur für die Umsetzung gemäß Verfahren (a) und(b) kann innerhalb eines größeren Bereiches variiert werden. Im allgemeinen arbeitet man bei Temperaturen zwischen -20°C und +150°C, vorzugsweise bei -5°C bis +120°C.

Die erfindungsgemäßen Verfahren (a) und (b) werden im allgemeinen bei Normaldruck durchgeführt.

Bei der Durchführung des erfindungsgemäßen Verfahrens (a) setzt man gewöhnlich äquimolare Mengen an Imidazol, Base und Senföl ein, und die auf diese Weise erhaltenen Verbindungen der Formel (III) werden gegebenenfalls nach Isolierung mit 1 Mol Halogenid der Formel (IV) umgesetzt.

Bei der Durchführung des erfindungsgemäßen Verfahrens (b) setzt man gewöhnlich äquimolare Mengen an Imidazol bzw. Triazol, Base und Dihalogenid der Formel (V) ein, und die auf diese Weise erhaltenen Verbindungen der Formel (VI) werden gegebenenfalls nach Isolierung mit 1 Mol Thiol der Formel (VII) bzw. dessen Salz und 1 Mol Base umgesetzt.

Die Aufarbeitung der Verbindungen der Formel (I) geschieht nach üblichen Methoden. Zu ihrer Charakterisierung dienen $^{1}$H-NMR-Spektren und/oder der Siedepunkt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe der Formel (I) in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die Wirkstoffe mit gutem Erfolg zur Bekämpfung von Pflanzenkrankheiten im Reisanbau, wie beispielsweise von Pyricularia oryzae und Pellicularia sasakii, von Getreidekrankheiten wie beispielsweise Braunrost an Weizen (Puccinia recondita), Mehltau (Erysiphe graminis), Cochliobolus sativus und Pyrenophora teres, von Apfelschorf (Venturia inaequalis), echtem Mehltau an Äpfeln, oder von Oomyceten eingesetzt werden.

Außerdem haben die erfindungsgemäßen Wirkstoffe eine breite fungizide Wirkung im Agarplattentest.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylaryl-polyglykol-ether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Verwendungsbeispiele :

In den nachfolgenden Verwendungsbeispielen werden die nachstehend angegebenen Verbindungen als Vergleichsverbindungen eingesetzt :

(A) 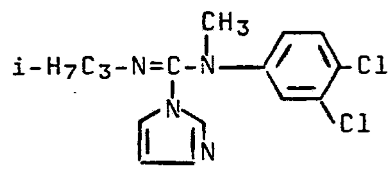

(B) 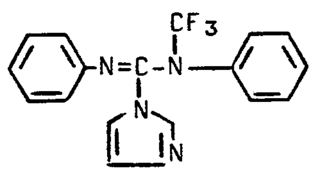

(C) 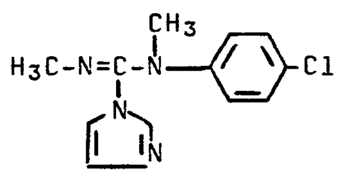

(D) 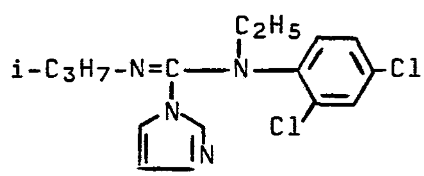

Beispiel A

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 1, 59, 12, 13, 14 und 15.

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1 und 28.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 17, 47 und 9.

Beispiel D

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 28 und 12.

Herstellungsbeispiele :

Beispiel 1 :

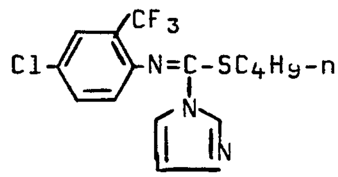

(Verfahren a)

Zu 68 g (1 Mol) Imidazol in 500 ml absolutem Tetrahydrofuran werden bei 20°C unter Kühlen 112,2 g (1 Mol) Kalium-tert.-butylat in 500 ml absolutem Tetrahydrofuran getropft. Die breiartige Suspension des Imidazol-Kaliumsalzes wird bei 0°C tropfenweise versetzt mit 237,6 g (1 Mol) 2-Trifluormethyl-4-chlorphenylsenföl in 500 ml absolutem Tetrahydrofuran. Nach der Zugabe entsteht eine klare Lösung, die bei 0°C versetzt wird mit 137 g (1 Mol) n-Butylbromid. Das Reaktionsgemisch wird 24 Stunden bei 20°C weiter gerührt. Anschließend wird eingeengt und der Rückstand mit Essigester und Wasser extrahiert. Die organische Phase wird eingeengt und das erhaltene Oel im Vakuum destilliert.

Man erhält 246 g (68 % der Theorie) N-[(n-Butylthio)-(imidazol-1-yl)-methyliden]-N-(4-chlor-2-trifluormethyl-phenyl)-amin vom Siedepunkt 155°C bei 0,1 Torr.

Beispiel 2 :

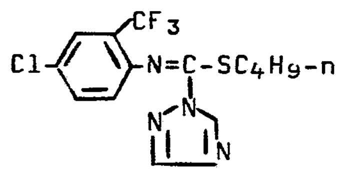

(Verfahren b)

α) Herstellung ohne Isolierung des Halogenids der Formel (VI) :

11,1 g (0,04 Mol) 4-Chlor-2-trifluormethyl-phenylisocyaniddichlorid in 40 ml Tetrahydrofuran werden mit 2,8 g (0,04 Mol) Triazol und 4,1 g (0,04 Mol) Triethylamin versetzt und eine Stunde bei 20°C gerührt. Danach werden 3,6 g (0,04 Mol) n-Butanthiol und 4,1 g (0,04 Mol) Triethylamin zugegeben und eine Stunde bei 20°C gerührt. Nach Entfernen des Lösungsmittels wird in 50 ml Essigester und 100 ml Wasser aufgenommen. Die organische Phase wird eingeengt und der Rückstand im Hochvakuum destilliert.

Man erhält eine Fraktion mit einem Siedebereich bis 140°C/0,5 Torr und eine zweite Fraktion mit einem Siedebereich > 140°C/0,5 Torr.

Die zweite Fraktion wird auf Kieselgel mit einem Ether/Petrolether-(1:1)-Gemisch chromatographiert. Das erhaltene Oel wird im Kugelrohrofen im Hochvakuum destilliert.

Man erhält 4,6 g (31 % der Theorie) N-[(n-Buthylthio)-(1,2,4-triazol-1-yl)-methyliden)-N-(4-chlor-2-trifluormethyl-phenyl)-amin vom Siedepunkt 150°C bei 0,5 Torr.

B) Herstellung nach Isolierung des Halogenids der Formel (VI) :

15,5 g (0,05 Mol) N-[(Chlor)-(1,2,4-triazol-1-yl)-methyliden]-N-(4-chlor-2-trifluormethyl-phenyl)-amin in 50 ml Tetrahydrofuran, 4,5 g (0,05 Mol) n-Butanthiol und 5,05 g (0,05 Mol) Triethylamin werden 25 Stunden bei 20°C gerührt. Das Lösungsmittel wird entfernt und der Rückstand wird in Essigester und Wasser aufgenommen. Die organische Phase wird vom Lösungsmittel befreit und anschließend im Hochvakuum gereinigt.

Man erhält 12,7 g (70 % der Theorie) N-[(n-Butylthio)-(1,2,4-triazol-1-yl)-methyliden]-N-(4-chlor-2-trifluormethylphenyl)-amin vom Siedepunkt 150°C bis 155°C bei 0,5 Torr.

Ausgangsprodukt der Formel (VI) :

Beispiel (VI-1) :

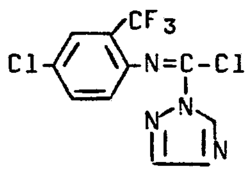

27.6 g (0,1 Mol) 4-Chlor-2-trifluormethyl-phenyliso-cyaniddichlorid, 6.9 g (0,1 Mol) Triazol und 13,8 g (0,1 Mol) Kaliumcarbonat in 100 ml Aceton werden 24 Stunden unter Rückfluß erhitzt. Anschließend wird das Reaktionsgemisch filtriert und vom Lösungsmittel befreit. Der Rückstand wird in Ether und Wasser aufgenommen. Die organische Phase wird nach Entfernen des Ethers mit 30 ml Petrolether verrührt, auf 0°C abgekühlt und filtriert.

Man erhält 15,8 g (51 % der Theorie) N-[(Chlor)-(1,2,4-triazol-1-yl)-methyliden]-N-(4-chlor-2-trifluormethyl-phenyl)-amin vom Schmelzpunkt 165 °C bis 166°C (Toluol).

Entsprechend Beispiel (VI-1) können die übrigen Verbindungen der Formel (VI) erhalten werden.

Analog den Beispielen 1 und 2 können nach den Verfahrensvarianten (a) und (b) die folgenden Verbindungen der allgemeinen Formel (I)

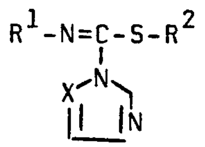

(I)

hergestellt werden:

**Tabelle 2**

| Beisp. Nr. | X | $R^1$ | $R^2$ | $^1H$-NMR($CDCl_3$): δ/ppm | |
|---|---|---|---|---|---|
| 3 | CH | $n-C_3H_7$ | $-H_2C-$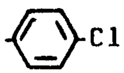$-Cl$ | 0,85 (t) | [ $C\boxed{H_3}CH_2-CH_2-N=$ ] |
| 4 | CH | $i-C_3H_7$ | $-H_2C-$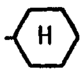 | 1,25 (d) | [ $-SC\boxed{H_2}-$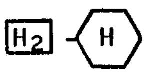 ] |
| 5 | CH | $n-C_6H_{13}$ | $-CH_3$ | 0,9 (t) | [ $C\boxed{H_3}(CH_2)_5-N=$ ] |
| 6 | CH | $n-C_6H_{13}$ | $-C_2H_5$ | 0,9 (t) | [ $C\boxed{H_3}(CH_2)_5-N=$ ] |
| 7 | CH | $n-C_6H_{13}$ | $-C_3H_7-n$ | 2,8 (t) | [ $-SC\boxed{H_2}CH_2CH_3$ ] |
| 8 | CH | $n-C_6H_{13}$ | $-C_3H_7-i$ | 0,95 (t) | [ $C\boxed{H_3}(CH_2)_5-N=$ ] |
| 9 | CH | $n-C_6H_{13}$ | $-C_4H_9-n$ | 2,8 (t) | [ $-SC\boxed{H_2}CH_2CH_2CH_3$ ] |
| 10 | CH | $n-C_6H_{13}$ | $-CH_2-CH=CH_2$ | 0,9 (t) | [ $C\boxed{H_3}(CH_2)_5-N=$ ] |
| 11 | CH | $n-C_6H_{13}$ | $-CH(CH_3)COOCH_3$ | 0,9 (t) | [ $C\boxed{H_3}(CH_2)_5-N=$ ] |
| 12 | CH | 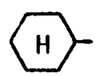 | $-CH_3$ | 2,5 (s) | [ $-SC\boxed{H_3}$ ] |
| 13 | CH | 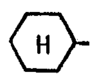 | $-C_2H_5$ | 2,8 (g) | [ $-SC\boxed{H_2}CH_3$ ] |
| 14 | CH | 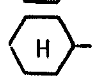 | $-C_3H_7-i$ | 1,25 (d) | [ $(C\boxed{H_3})_2CHS-$ ] |

**Tabelle 2** - Fortsetzung

| Beisp. Nr. | X | $R^1$ | $R^2$ | $^1H$-NMR($CDCl_3$): $\delta$/ppm | | |
|---|---|---|---|---|---|---|
| 15 | CH | 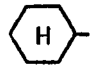 | $-C_4H_9-n$ | 0,9 | (t) | [ C $\boxed{H_3}$ $CH_2CH_2CH_2S-$ ] |
| 16 | CH | 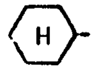 | $-CH_2-$ 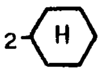 | 2,6 | (d) | [ -SC $\boxed{H_2}$ 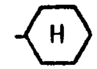 ] |
| 17 | CH | 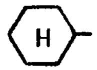 | $-CH_2-CH=CH_2$ | 3,2 | (d) | [ -SC $\boxed{H_2}$ $CH=CH_2$ ] |
| 18 | CH | $CH_2=CH-CH_2-$ | $-H_2C$ 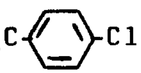 Cl | 4,0 | (s) | [ -SC $\boxed{H_2}$ 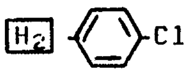 Cl ] |
| 19 | CH | Cl 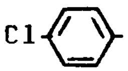 $C_2H_4-$ | $-C_4H_9-n$ | 0,8 | (t) | [ C $\boxed{H_3}$ $(CH_2)_3-$ ] |
| 20 | CH | Cl 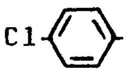 $C_2H_4-$ | $-C_5H_{11}-n$ | 0,9 | (t) | [ C $\boxed{H_3}$ $(CH_2)_4-$ ] |
| 21 | CH | Cl 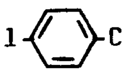 $C_2H_4-$ | $-C_6H_{13}-n$ | 0,9 | (t) | [ C $\boxed{H_3}$ $(CH_2)_5-$ ] |
| 22 | CH | Cl 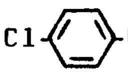 $C_2H_4-$ | $-C_{12}H_{25}-n$ | 0,9 | (t) | [ C $\boxed{H_3}$ $(CH_2)_{11}-$ ] |
| 23 | CH | Cl 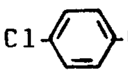 $C_2H_4-$ | 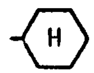 | 3,9 | (t) | [ $-CH_2C$ $\boxed{H_2}$ N= ] |
| 24 | CH | Cl 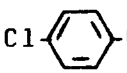 $C_2H_4-$ | 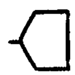 | 3,9 | (t) | [ $-CH_2C$ $\boxed{H_2}$ N= ] |
| 25 | CH | Cl 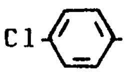 $C_2H_4-$ | $-H_2C$ 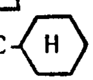 | 2,5 | (d) | [ -SC $\boxed{H_2}$ 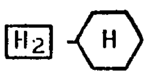 ] |

Tabelle 2 -Fortsetzung

| Beisp. Nr. | X | $R^1$ | $R^2$ | $^1$H-NMR($CDCl_3$): δ/ppm | |
|---|---|---|---|---|---|
| 26 | CH | 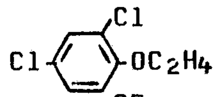 | $-C_4H_9$-n | 2,8 (t) | [ -SC $H_2$ $CH_2CH_2CH_3$ ] |
| 27 | CH | 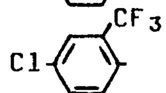 | $-CH_3$ | 2,3 (s) | [ C $H_3$ S- ] |
| 28 | CH | 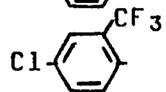 | $-C_2H_5$ | 1,2 (t) | [ C $H_3$ $CH_2S$- ] |
| 29 | CH | 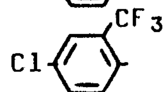 | $-C_3H_7$-n | 0,9 (t) | [ C $H_3$ $CH_2CH_2S$- ] |
| 30 | CH | 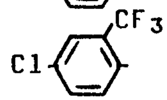 | $-C_3H_7$-i | 1,25 (d) | [ (C $H_3$ )$_2$CHS- ] |
| 31 | CH | 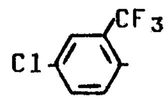 | $-C_2H_4$- 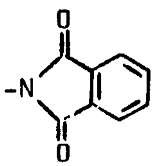 | 3,3 (t) | [ -SC $H_2$ $CH_2$- ] |
| 32 | CH | 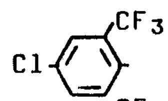 | $-C_4H_9$-i | 2,75 (d) | [ -SC $H_2$ $CH(CH_3)_2$ ] |
| 33 | CH | 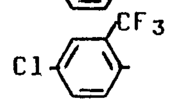 | $-C_4H_9$-sec | 0,85 (t) | [ C $H_3$ $-CH_2-CH(CH_3)-S-$ ] |

**Tabelle 2** - Fortsetzung

| Beisp. Nr. | X | $R^1$ | $R^2$ | $^1H$-NMR($CDCl_3$): $\delta$/ppm | |
|---|---|---|---|---|---|
| 34 | CH | 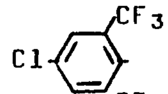 | $-C_4H_9$-tert. | 1,45 (s) | $[(C\boxed{H_3})_3CS-]$ |
| 35 | CH | 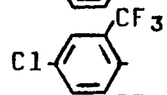 | $-C_5H_{11}$-n | 2,9 (t) | $[CH_3(CH_2)_3C\boxed{H_2}S-]$ |
| 36 | CH | 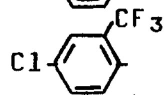 | $-C_5H_{11}$-sec. | 0,9 (t) | $[C\boxed{H_3}(CH_2)_2CH(CH_3)-S-]$ |
| 37 | CH | 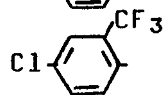 | $-C_6H_{13}$-n | 2,9 (t) | $[CH_3(CH_2)_4C\boxed{H_2}S-]$ |
| 38 | CH | 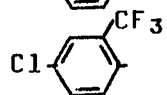 | $-C_7H_{15}$-n | 2,9 (t) | $[CH_3(CH_2)_5C\boxed{H_2}S-]$ |
| 39 | CH | 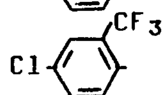 | $-C_7H_{15}$-sec. | 0,9 (t) | $[C\boxed{H_3}(CH_2)_4CH(CH_3)S-]$ |
| 40 | CH | 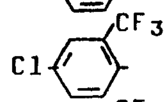 | $-C_8H_{17}$-n | 2,9 (t) | $[CH_3(CH_2)_6C\boxed{H_2}S-]$ |
| 41 | CH | 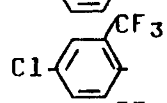 | $-CH(C_3H_7\text{-n})(C_4H_9\text{-n})$ | 0,7-8,2 (m) | [ arom. H; heteroarom. H] |
| 42 | CH | 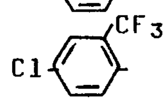 | $-C_9H_{19}$-n | 2,9 (t) | $[CH_3(CH_2)_7C\boxed{H_2}S-]$ |

**Tabelle 2** - Fortsetzung

| Beisp. Nr. | X | $R^1$ | $R^2$ | $^1H$-NMR($CDCl_3$): $\delta$/ppm | |
|---|---|---|---|---|---|
| 43 | CH | 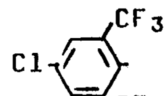 | $-C_{12}H_{25}-n$ | 2,9 (t) | [ $CH_3(CH_2)_{10}C\boxed{H_2}S-$ ] |
| 44 | CH | 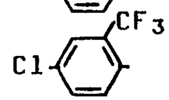 | 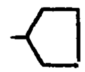 | 3,3-3,8 (m) | 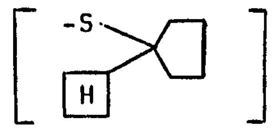 |
| 45 | CH | 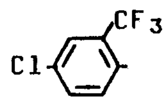 | 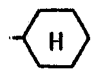 | 3,0-3,5 (m) | 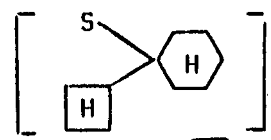 |
| 46 | CH | 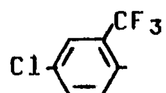 | $-CH_2-$ 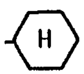 | 2,75 (d) | [ $-SC\boxed{H_2}-$ 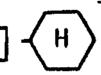 ] |
| 47 | CH | 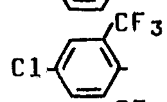 | $-CH_2-CH=CH_2$ | 3,45 (d) | [ $-SC\boxed{H_2}CH=CH_2$ ] |
| 48 | CH | 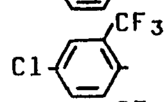 | $-CH_2-CH=CH-CH_3$ | 1,60 (d) | [ $C\boxed{H_3}-CH=CH-CH_2-$ ] |
| 49 | CH | 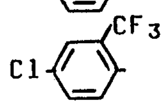 | $-CH_2-CH=\underset{Cl}{C}-CH_3$ | 2,1 (s) | [ $C\boxed{H_3}-C(Cl)-$ ] |
| 50 | CH | 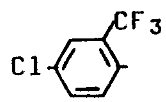 | $-CH_2-\underset{CH_3}{C}=CH_2$ | 1,75 (s) | [ $C\boxed{H_3}\,C(=CH_2)CH_2S-$ ] |

<u>Tabelle 2</u> - Fortsetzung

| Beisp. Nr. | X | $R^1$ | $R^2$ | $^1$H-NMR($CDCl_3$): δ/ppm | |
|---|---|---|---|---|---|
| 51 | CH | Cl-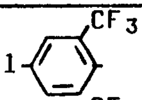 | $-CH_2-C{\equiv}CH$ | 2,2 (t) | [ $C\boxed{H}{\equiv}C-CH_2-$ ] |
| 52 | CH | Cl-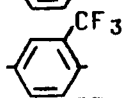 | $-C_2H_4-O-C_2H_5$ | 1,1 (t) | [ $C\boxed{H_3}\ CH_2O-$ ] |
| 53 | CH | Cl-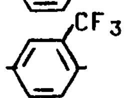 | $-CH_2-CH(OC_2H_5)_2$ | 3,2 (d) | [ $-SC\boxed{H_2}\ CH(OC_2H_5)_2$ ] |
| 54 | CH | Cl-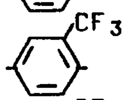 | $-C(CH_3)_2-COCH_3$ | 1,7 (s) | [ $-C(C\boxed{H_3}\ _2-CO-CH_3$ ] |
| 55 | CH | Cl-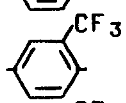 | $-C_2H_4-O-COCH_3$ | 2,0 (s) | [ $C\boxed{H_3}\ -CO-$ ] |
| 56 | CH | Cl-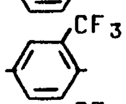 | $-(CH_2)_4-O-COCH_3$ | 2,0 (s) | [ $C\boxed{H_3}\ -CO-O-$ ] |
| 57 | CH | Cl-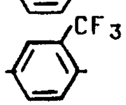 | $-C_2H_4-COOCH_3$ | 2,0 (s) | [ $C\boxed{H_3}\ OCO-$ ] |
| 58 | CH | Cl-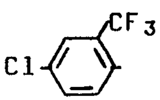 | 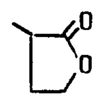 | 2,1-2,9 (m) | [ 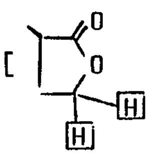 ] |

**Tabelle 2** - Fortsetzung

| Beisp. Nr. | X | $R^1$ | $R^2$ | $^1H$-NMR($CDCl_3$): δ/ppm | |
|---|---|---|---|---|---|
| 59 | CH | 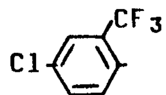 | $-CH(CH_3)COOCH_3$ | 1,65 (d) | [ $-CH(C\boxed{H_3})COOCH_3$ ] |
| 60 | N | 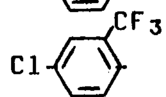 | $-CH_2-CH=CH_2$ | 3,7 (d) | [ $-S-C\boxed{H_2}-CH=CH_2$ ] |
| 61 | N | 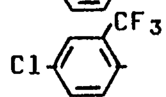 | $C_5H_{11}$-n | 3,05 (t) | [ $-SC\boxed{H_2}(CH_2)_3CH_3$ ] |
| 62 | N | 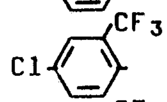 | $C_8H_{17}$-n | 3,0 (t) | [ $-SC\boxed{H_2}(CH_2)_6CH_3$ ] |
| 63 | N | 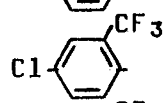 | 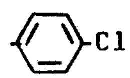 | 7,9 (s)<br>8,4 (s) | [ Triazol-H ] |
| 64 | N | 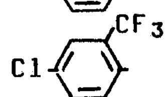 | 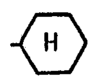 | 8,0 (s)<br>8,5 (s) | [ Triazol-H ] |
| 65 | N | 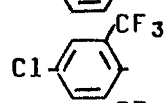 | $C_6H_{13}$-n | 3,0 (t) | [ $-SC\boxed{H_2}(CH_2)_4CH_3$ ] |
| 66 | N | 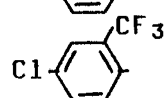 | $C_{12}H_{25}$-n | 3,0 (t) | [ $-SC\boxed{H_2}(CH_2)_{10}CH_3$ ] |
| 67 | N | 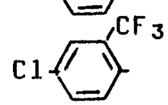 | 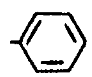 | 8,0 (s)<br>8,5 (s) | [ Triazol-H ] |

**Tabelle 2** - Fortsetzung

| Beisp. Nr. | X | $R^1$ | $R^2$ | $^1$H-NMR($CDCl_3$): δ/ppm | |
|---|---|---|---|---|---|
| 68 | N | 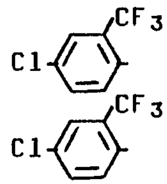 | $-C(CH_3)_3$ | 1,55 (s) | [ (C H3 )$_3$C- ] |
| 69 | N | | 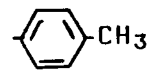 | 2,25 (s) | [ 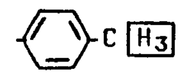 ] |

Tabelle 2 - Fortsetzung

| Beisp. Nr. | X | $R^1$ | $R^2$ | $^1H$-NMR ($CDCl_3$): $\delta$/ppm |
|---|---|---|---|---|
| 70 | CH | $-CH_2CH_2CH_2OCH_3$ | $-C_4H_{9}$-n | 3,3 (s) [$\overline{C}\boxed{H_3}O(CH_2)_3-N=$] |
| 71 | CH | $-CH_2CH_2CH_2OCH_3$ | 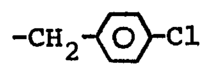 | 3,3 (s) [$\overline{C}\boxed{H_3}O(CH_2)_3-N=$] |
| 72 | CH | $-CH_2CH_2CH_2OCH_3$ | 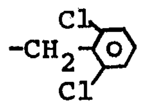 | 3,3 (s) [$\overline{C}\boxed{H_3}O(CH_2)_3-N=$] |
| 73 | N | 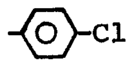 | $-(CH_2)_5-CH_3$ | 2,9 (t) [$\overline{-}S-C\boxed{H_2}(CH_2)_4CH_3$] |
| 74 | N | 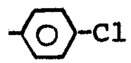 | $-(CH_2)_3-CH_3$ | 2,9 (t) [$\overline{-}S-C\boxed{H_2}(CH_2)_2CH_3$] |
| 75 | N | 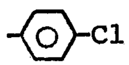 | $-CH_2-CH=CH_2$ | 3,65 (d) [$\overline{-}S-C\boxed{H_2}CH=CH_2$] |
| 76 | N | 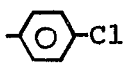 | 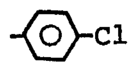 | Fp: 100-107°C |
| 77 | N | 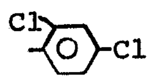 | $-CH_2-CH=CH_2$ | 3,5 (d) [$\overline{-}S-C\boxed{H_2}CH=CH_2$] |
| 78 | N | 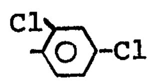 | 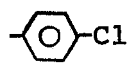 | Fp: 110-112°C |
| 79 | N | 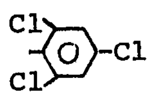 | 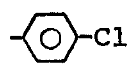 | Fp: 128-130°C |

**Tabelle 2** - Fortsetzung

| Beisp. Nr. | X | $R^1$ | $R^2$ | $^1H$-NMR ($CDCl_3$): $\delta$ /ppm |
|---|---|---|---|---|
| 80 | N | 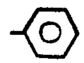 | 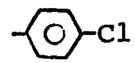 | Fp: 78-81°C |
| 81 | N | 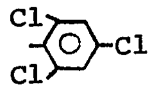 | $-CH_2-CH=CH_2$ | 3,8 (d) /-S-C $H_2$ $CH=CH_2$-/ |
| 82 | N | 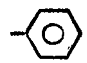 | $-C_4H_9$-n | 2,9 (t) /-S-C $H_2$ $CH_2CH_2CH_3$-/ |
| 83 | N | 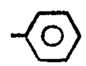 | $-CH_2-CH=CH_2$ | 3,55 (d) /-S-C $H_2$ $CH=CH_2$-/ |
| 84 | CH | 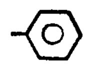 | $-CH_2-CH=CH_2$ | 3,4 (d) /-S-C $H_2$ $CH=CH_2$-/ |
| 85 | CH | 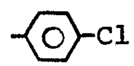 | $-CH_2CH_2OCH_2CH_3$ | 1,1 (t) /-S-$CH_2-CH_2OCH_2C$ $H_3$-/ |
| 86 | CH | 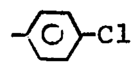 | $-CH_2-CH=CH_2$ | 3,7 (d) /-S-C $H_2$ $CH=CH_2$-/ |
| 87 | CH | 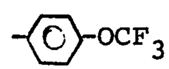 | $-CH_2-CH=CH_2$ | 3,4 (d) /-S-C $H_2$ $CH=CH_2$-/ |
| 88 | CH | 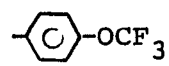 | $-C_4H_9$-n | 2,8 (t) /-S-C $H_2$ $CH_2CH_2CH_3$-/ |
| 89 | CH | 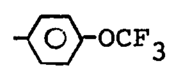 | $-(CH_2)_{11}CH_3$ | 2,8 (t) /-S-C $H_2$ $(CH_2)_{10}CH_3$-/ |

Tabelle 2 - Fortsetzung

| Beisp. Nr. | X | $R^1$ | $R^2$ | $^1H$-NMR ($CDCl_3$): $\delta$ /ppm |
|---|---|---|---|---|
| 90 | CH | $-(CH_2)_{11}CH_3$ | $-CH_2-CH=CH_2$ | 3,6 (t) [$\bar{C}H_3(CH_2)_{10}C\boxed{H_2}-N=$] |
| 91 | CH | 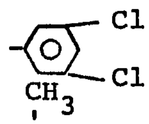 | $-CH_2-CH=CH_2$ | 3,4 (d) [$-S-C\boxed{H_2}CH=CH_2-$] |
| 92 | CH | $-\overset{CH_3}{CH}-(CH_2)_5CH_3$ | $-CH_3$ | 0,9 (t) [$\bar{C}\boxed{H_3}(CH_2)_5\overset{CH_3}{CH}-N=$] |
| 93 | CH | $-\overset{CH_3}{CH}-(CH_2)_5CH_3$ | $-C_4H_9-n$ | 2,8 (t) [$-S-C\boxed{H_2}CH_2CH_2CH_3-$] |
| 94 | CH | $-\overset{CH_3}{CH}-(CH_2)_5-CH_3$ | $-(CH_2)_{11}CH_3$ | 2,8 (t) [$-S-C\boxed{H_2}(CH_2)_{10}CH_3-$] |
| 95 | CH | $-(CH_2)_{11}CH_3$ | $-(CH_2)_{11}CH_3$ | 2,7 (t) [$-S-C\boxed{H_2}(CH_2)_{10}CH_3-$] |
| 96 | CH | $-(CH_2)_{11}CH_3$ | $-C_6H_{13}-n$ | 2,7 (t) [$-S-C\boxed{H_2}(CH_2)_4CH_3-$] |
| 97 | CH | $-(CH_2)_{11}CH_3$ | $-C_4H_9-n$ | 2,7 (t) [$-S-C\boxed{H_2}CH_2CH_2CH_3-$] |
| 98 | CH | $-(CH_2)_{11}CH_3$ | $-CH_3$ | 3,7 (t) [$\bar{C}H_3(CH_2)_{10}C\boxed{H_2}-N=$] |
| 99 | CH | 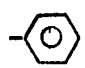 | $-C_4H_9-n$ | 2,7 (t) [$-S-C\boxed{H_2}(CH_2)_2CH_3-$] |

<u>Tabelle 2</u> - Fortsetzung

| Beisp. Nr. | X | $R^1$ | $R^2$ | $^1H$-NMR ($CDCl_3$): $\delta$/ppm |
| --- | --- | --- | --- | --- |
| 100 | CH | 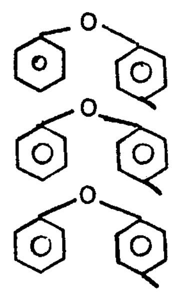 | $-CH_2CH_2OCH_3$ | 3,3 (s) /‾S-$CH_2CH_2OC$ [$H_3$] _7 |
| 101 | CH | | $-C_4H_9$-n | 2,7 (t) /‾S-C [$H_2$] $CH_2CH_2CH_3$ _7 |
| 102 | CH | | $-CH_2-CH=CH_2$ | 3,4 (d) /‾S-C [$H_2$] $CH=CH_2$ _7 |

Patentansprüche

1. Iminomethyl-azolyl-Derivate der allgemeinen Formel (I)

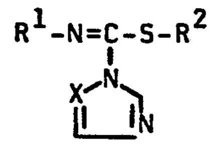

(I)

in welcher

X für Stickstoff oder eine CH-Gruppe steht,

$R^1$ für geradkettiges oder verzweigtes Alkyl, Alkenyl, Alkoxyalkyl, gegebenenfalls substituiertes Cycloalkyl, gegebenenfalls im Phenylteil substituiertes Phenyl, Phenylalkyl oder Phenoxyalkyl steht, und

$R^2$ für gegebenenfalls substituiertes, geradkettiges oder verzweigtes Alkyl, für Alkenyl, Alkinyl, Halogenalkyl, Halogenalkenyl, Cyano, gegebenenfalls substituiertes Benzyl oder Phenyl, sowie für einen gegebenenfalls durch Sauerstoff ung/oder eine Ketogruppe unterbrochenen Cycloalkyl-Rest steht,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen,

und deren Säureadditionssalze und Metallsalzkomplexe.

2. Verbindungen der allgemeinen Formel (I) in Anspruch 1, wobei

X für Stickstoff oder eine CH-Gruppe steht,

$R^1$ für geradkettiges oder verzweigtes Alkyl und Alkoxyalkyl mit jeweils 1 bis 12 Kohlenstoffatomen in den Alkylteilen, für Alkenyl mit bis zu 4 Kohlenstoffatomen, für gegebenenfalls einfach oder mehrfach, gleich oder verschieden durch Halogen und/oder Alkyl substituiertes Cycloalkyl mit 3 bis 8 Kohlenstoffatomen, für gegebenenfalls einfach oder mehrfach, gleich oder verschieden im Phenylteil substituiertes Phenyl, Phenylalkyl und Phenoxyalkyl, mit jeweils 1 bis 6 Kohlenstoffatomen im Alkylteil steht, wobei als jeweilige Phenylsubstituenten infrage kommen:
Halogen, Alkyl, Alkoxy und Alkylthio mit jeweils 1 bis 4 Kohlenstoffatomen im Alkylteil, Halogenalkyl, Halogenalkoxy und Halogenalkylthio mit jeweils 1 bis 4 Kohlenstoffatomen im Alkylteil und 1 bis 5 gleichen oder verschiedenen Halogenatomen, wie Fluor, Chlor und/oder Brom; und/oder Phenoxy;

$R^2$ für gegebenenfalls einfach oder mehrfach, gleich oder verschieden substituiertes, geradkettiges oder verzweigtes Alkyl mit 1 bis 12 Kohlenstoffatomen steht, wobei als Alkylsubstituenten bevorzugt infrage kommen :

ein Phthalimido-Rest; Cyano; Halogen, wie Fluor, Chlor, Brom und/oder Iod; eine gegebenenfalls durch Sauerstoff unterbrochene Cycloalkylgruppierung mit 2 bis 6 Kohlenstoffatomen; und/oder die Gruppierungen $R^3O$-, $R^3S$-, $R^3$-CO-O, $R^3O$-CO, $R^3$-CO-, $R^3O$-CO-$(CH_2)_n$-CO-, $(R^4O)(R^5O)$-$CR^6$ und $R^7R^8N$-CO-, in welcher

$R^3$, $R^4$ und $R^5$ gleich oder verschieden sind und für geradkettiges oder verzweigtes Alkyl mit 1 bis 6 Kohlenstoffatomen oder für gegebenenfalls durch Fluor, Chlor, Methyl, Methoxy, Methylthio, Trifluormethyl, Trifluormethoxy und/oder Trifluormethylthio substituiertes Phenyl stehen, oder

$R^4$ und $R^5$ gemeinsam für einen gegebenenfalls durch Fluor und/oder Chlor substituierten Alkandiyl-Rest mit 2 oder 3 Kohlenstoffatomen stehen,

n für die Zahlen 0 oder 1 steht,

$R^6$, $R^7$ und $R^8$ gleich oder verschieden sind und für Wasserstoff, geradkettiges oder verzweigtes Alkyl mit 1 bis 4 Kohlenstoffatomen oder für gegebenenfalls durch Fluor, Chlor, Methyl, Methoxy, Methylthio, Trifluormethyl, Trifluormethoxy und/oder Trifluormethylthio substituiertes Phenyl stehen; und

$R^2$ weiterhin für Alkenyl und Alkinyl mit 3 bis 6 Kohlenstoffatomen steht; für Halogenalkenyl mit 3 bis 6 Kohlenstoffatomen im Alkenylteil und 1 bis 3 Halogenatomen, wie insbesondere Chlor, Brom und/oder Iod steht; für Cyano oder für gegebenenfalls substituiertes Benzyl oder Phenyl steht, wobei als Phenylsubstituenten vorzugsweise die Substituenten infrage kommen, die bei der Beschreibung von $R^1$ als Phenylsubstituenten genannt worden sind; sowie für eine gegebenenfalls durch Sauerstoff und/oder eine Ketogruppe unterbrochene Cycloalkylgruppe mit 3 bis 6 Kohlenstoffatomen steht,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen.

3. Verbindungen der allgemeinen Formel (I) in Anspruch 1, wobei

X für Stickstoff oder die CH-Gruppe steht,

$R^1$ für Alkyl mit 1 bis 12 Kohlenstoffatomen und für Alkenyl mit bis zu 4 Kohlenstoffatomen steht, ferner für Cycloalkyl 5 bis 6 Kohlenstoffatomen steht, wobei letzteres durch 1 bis 3 Alkylgruppen mit bis zu 3 Kohlenstoffatomen substituiert sein kann, und schließlich für Phenyl, Phenylalkyl und Phenoxyalkyl mit jeweils bis zu 4 Kohlenstoffatomen im Alkylteil steht, wobei die drei letztgenannten Gruppen im Phenylteil ein- bis dreifach substituiert sein können durch Halogen, Alkyl, Alkoxy und Alkylthio mit jeweils 1 bis 3 Kohlenstoffatomen, und/oder durch Halogenalkyl mit bis zu 2 Kohlenstoff- und bis zu 5 Halogenatomen, wie insbesondere durch Trifluormethyl, und

$R^2$ für Alkenyl und Alkinyl mit 3 bis 4 Kohlenstoffatomen steht, wobei die beiden letztgenannten Reste 1 bis 3 Halogenatome tragen können, weiterhin für Cyano steht, für Cyclohexylmethyl oder für Phenyl, wobei der letztgenannte Rest durch die bei $R^1$ genannten Phenylsubstituenten substituiert sein kann, und schließlich für eine Cycloalkylgruppe mit 3 bis 6 Kohlenstoffatomen steht, in welche Sauerstoff oder eine Carbonylgruppe in das Ringsystem eingefügt sein kann, weiterhin kann noch für den Fall, daß

a) nicht gleichzeitig
   X für eine CH-Gruppe und
   $R^1$ für durch Halogen und/oder Alkyl substituiertes Phenyl steht,
   $R^2$ noch zusätzlich stehen für primäres, sekundäres

oder tertiäres Alkyl mit bis zu 12 Kohlenstoffatomen steht, welches substituiert sein kann durch Halogen, Cyano, einen Phthalimido-Rest, durch Alkoxy- und Alkylthio-Reste mit jeweils bis zu 3 Kohlenstoffatomen, durch Alkylcarbonyl, Alkoxycarbonyl und Alkylcarbonyloxy mit jeweils bis zu 4 Kohlenstoffatomen in den Alkylteilen, und schließlich kann noch für den Fall, daß

b) nicht gleichzeitig
X für Stickstoff oder eine CH-Gruppe und
$R^1$ für gegebenenfalls durch Halogen, Alkyl, Alkoxy und/oder Trifluormethyl substituiertes Phenyl steht,
$R^2$ noch zusätzlich für Benzyl stehen, welches durch die bei $R^1$ genannten Phenylsubstituenten im Phenylteil substituiert sein kann.

4. Verfahren zur Herstellung von Iminomethyl-azolyl-Derivaten der allgemeinen Formel (I)

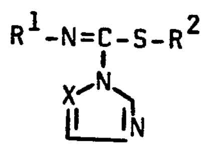

(I)

in welcher

X für Stickstoff oder eine CH-Gruppe steht,

$R^1$ für geradkettiges oder verzweigtes Alkyl, Alkenyl, Alkoxyalkyl, gegebenenfalls substituiertes Cycloalkyl, gegebenenfalls im Phenylteil substituiertes Phenyl, Phenylalkyl oder Phenoxyalkyl steht, und

$R^2$ für gegebenenfalls substituiertes, geradkettiges oder verzweigtes Alkyl, für Alkenyl, Alkinyl, Halogenalkyl, Halogenalkenyl, Cyano, gegebenenfalls substituiertes Benzyl oder Phenyl, sowie für einen gegebenenfalls durch Sauerstoff und/oder eine Ketogruppe unterbrochenen Cycloalkyl-Rest steht,

wobei jedoch nicht gleichzeitig

a) X für eine CH-Gruppe,

$R^1$ für durch Halogen und/oder Alkyl substituiertes Phenyl und

$R^2$ für Alkyl stehen dürfen, oder

b) X für Stickstoff oder eine CH-Gruppe,

$R^1$ für gegebenenfalls durch Halogen, Alkyl, Alkoxy und/oder Trifluormethyl substituiertes Phenyl und

$R^2$ für gegebenenfalls durch Halogen, Alkyl, Alkoxy und/oder Trifluormethyl substituiertes Benzyl stehen dürfen

dadurch gekennzeichnet, daß man

a) für den Fall, daß X für eine CH-Gruppe steht, Senföle der Formel (II)

$$R^1-N=C=S \qquad (II)$$

in welcher

$R^1$ die oben angegebene Bedeutung hat,

mit Imidazol, in Gegenwart einer starken Base und gegebenenfalls in Gegenwart eines Verdünnungsmittels umsetzt, und die auf diese Weise erhaltenen Salze der Formel (III)

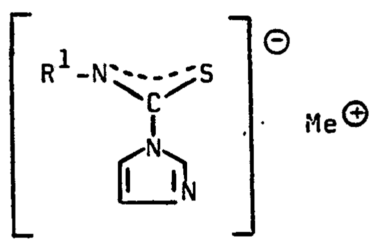

(III)

in welcher

$R^1$ die oben angegebene Bedeutung hat und

Me für ein Äquivalent eines Alkyli- oder Erdalkalimetallatoms steht,

gegebenenfalls isoliert und mit Halogeniden der Formel (IV)

$$R^2Hal \qquad (IV)$$

in welcher
$R^2$ die oben angegebene Bedeutung hat und
Hal für Halogen steht,

gegebenenfalls in Gegenwart eines Verdünnungsmittels umsetzt;

oder daß man

b) für den Fall, daß X für eine CH-Gruppe oder für Stickstoff steht,
Isocyanid-dihalogenide der Formel (V)

$$R^1\text{-}N{=}C(Hal^1)_2 \qquad (V)$$

in welcher
$R^1$ die oben angegebene Bedeutung hat, und
$Hal^1$ für Halogen, wie Chlor oder Brom steht,

mit Imidazol bzw. Triazol, in Gegenwart eines Säureakzeptors und gegebenenfalls in Gegenwart eines Verdünnungsmittels umsetzt und die auf diese Weise erhaltenen Verbindungen der Formel (VI)

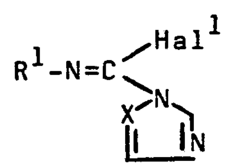

(VI)

in welcher

$R^1$, X und $Hal^1$ die oben angegebenen Bedeutungen haben,

gegebenenfalls isoliert und mit Thiolen der Formel (VII)

$$R^2S-Y \qquad (VII)$$

in welcher

$R^2$ die oben angegebene Bedeutung hat und

Y für Wasserstoff oder ein Aequivalent eines Alkali- oder Erdalkali-metallatoms steht,

gegebenenfalls in Gegenwart eines Säureakzeptors und gegebenenfalls in Gegenwart von Verdünnungsmitteln umsetzt, und weiterhin gegebenenfalls an die auf diese Weise erhaltenen Verbindungen der Formel (I) anschließend eine Säure oder ein Metallsalz addiert.

5. Fungizide Mittel, gekennzeichnet durch einen Gehalt an mindestens einem Iminomethyl-azolyl-Derivat der Formel (I) in Ansprüchen 1 und 4.

6. Verfahren zur Bekämpfung von Pilzen, dadurch gekennzeichnet, daß man Iminomethyl-azolyl-Derivate der Formel (I) in Ansprüchen 1 und 4, auf Pilze oder ihren Lebensraum einwirken läßt.

7. Verwendung von Iminomethyl-azolyl-Derivaten der Formel (I) in Ansprüchen 1 und 4 als Pflanzenschutzmittel.

8. Verwendung von Iminomethyl-azolyl-Derivaten der Formel (I) in Ansprüchen 1 und 4, zur Bekämpfung von Pilzen.

9. Verfahren zur Herstellung von fungiziden Mitteln, dadurch gekennzeichnet, daß man Iminomethyl-azolyl-Derivate der Formel (I) in Ansprüchen 1 und 4 mit Streckmitteln und/oder oberflächenaktiven Mitteln vermischt.

10. Salze der Formel

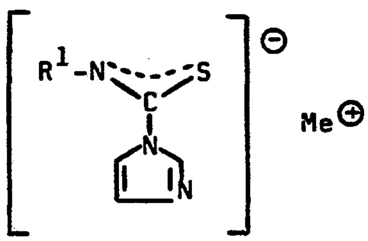 (III)

in welcher

$R^1$ für geradkettiges oder verzweigtes Alkyl, Alkenyl, Alkoxyalkyl, gegebenenfalls substituiertes Cycloalkyl, gegebenenfalls im Phenylteil substituiertes Phenyl, Phenylalkyl oder Phenoxyalkyl steht, und

Me für ein Äquivalent eines Alkali- oder Erdalkalimetallatoms steht.